# EUROPEAN PATENT APPLICATION

(11) **EP 2 189 458 A1**
(43) Date of publication of application: **26.05.2010**
(21) Application number: 09252511.2
(22) Date of filing: 29.10.2009
(51) Int. Cl.: C07D 405/14, A61K 31/47, A61P 3/00

(54) **Aryl amide compound as an acetyl coenzyme A carboxylase inhibitor**

(30) Priority: 30.10.2008 US 109746 P
(71) Applicant: Janssen Pharmaceutica, N.V., 2340 Beerse (BE)
(72) Inventor: Player, Mark R., Phoenixville, PA 19460 (US); Liu, Jian, Manalapan, NJ 07726 (US)
(74) Representative: Warner, James Alexander

(57) **Abstract**

An aryl amide compound of Formula (I): 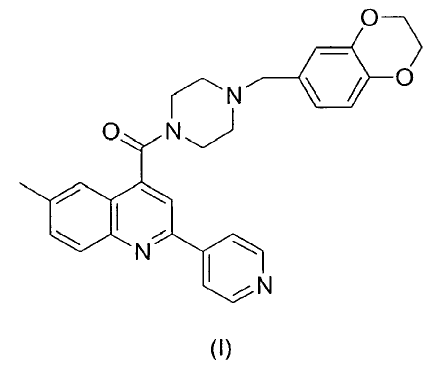 or a pharmaceutically acceptable form thereof is disclosed. The compound is an ACC inhibitor and therefore useful in treating an ACC-mediated condition.

## Description

### FIELD OF INVENTION

The present invention relates to an aryl amide compound useful as an acetyl coenzyme A carboxylase (ACC) inhibitor. More particularly, the present invention relates to a quinoline aryl substituted amide ACC inhibitor compound and methods of preparation and use thereof.

### BACKGROUND OF THE INVENTION

Acetyl coenzyme A (CoA) carboxylase (ACC) is an enzyme that carboxylates acetyl CoA to produce malonyl CoA. ACC has two isozymes: ACC1 and ACC2, both of which produce malonyl CoA. Malonyl CoA may be a starting material for long-chain fatty acids or triglycerides and may negatively control carnitine palmitoyl transferase-1 (CPT-1), which participates in oxidative decomposition of fatty acids. The isozyme ACC1 exists in cytoplasm and is considered a rate-limiting enzyme in the biosynthesis of long-chain fatty acids. ACC2 exists predominately in mitochondria and may participate principally in oxidation of fatty acids. Compounds capable of inhibiting either ACC1 and/or ACC2 are expected not only to inhibit synthesis of fatty acids but also to reduce accumulated fat.

High cellular fat and fatty acids, as well as malonyl CoA generated by ACC2, induce insulin resistance in animals and play an important role in type-2 diabetes. As well, an excess of accumulated fat may cause, for example, insulin resistance, diabetes, hypertension, hyperlipidemia and obesity. Moreover, diabetes often causes cardiac angina, heart failure, stroke, claudication, retinopathy, eyesight failure, renal failure, neuropathy, skin ulcer and infectious diseases. A combination of these factors, therefore, may lead to a higher risk of arteriosclerosis and metabolic syndrome.

Furthermore, hypertriglyceridemia (i.e., obesity) leads to a higher risk of, for example, pancreatitis, hepatic dysfunction, emmeniopathy, arthritis, gout, cholecystitis, gastroesophageal reflux, Pickwickian syndrome, and sleep apnea syndrome. Since tumor cells generally show an increased synthesis of fatty acids, and it is reported that some fatty acid synthesis inhibitors exhibit a cell growth-inhibiting effect and may be useful in the treatment of cancers, such as, breast, uterine, ovarian, colon and prostate cancers.

Accordingly, ACC inhibitors are expected to be useful for the treatment of disorders such as hyperlipidemia, dyslipidemia, hepatic steatosis, hepatic dysfunction, non-alcoholic fatty liver disease, obesity, diabetes, insulin resistance, metabolic syndrome, arteriosclerosis, hypertension, cardiac angina, heart failure, cardiac infarction, stroke, claudication, retinopathy, eyesight failure, renal failure, electrolyte abnormality, neuropathy, skin ulcer, bulimia, pancreatitis, emmeniopathy, arthritis, gout, cholecystitis, gastroesophageal reflux, Pickwickian syndrome, sleep apnea syndrome, infectious diseases, neoplasia, impaired glucose tolerance, pre-diabetes, hypertriglyceridemia, Cushings Syndrome, lipodystrophy, anemia, nephropathy or cancers (such as, breast, uterine, ovarian, colon and prostate cancers).

United States Patents 5,097,044, 5,210,232 and 5,362,872; United States Patent Publications US2006/0178400 and US2006/0270686; and, International Publications WO 1998/034920, WO 2003/059871, WO 2003/059886, WO 2003/072197, WO 2003/094912, WO 2007/011809, WO 2007/011811 and WO 2007/013691 describe ACC inhibitor compounds.

There still remains a need for ACC inhibitor compounds that have pharmacokinetic and pharmacodynamic properties suitable for use as human pharmaceuticals.

### SUMMARY OF THE INVENTION

The present invention relates to a quinoline aryl substituted amide compound of Formula (I):

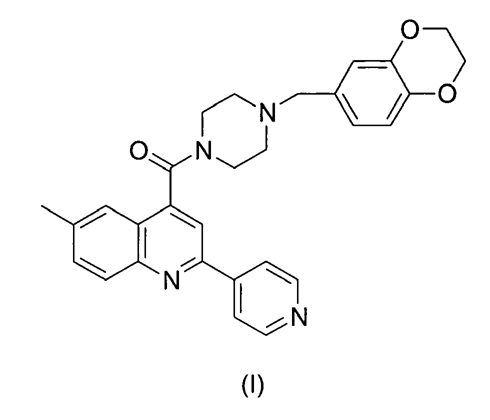

The compound of the present invention is an ACC inhibitor which is useful in treating an ACC mediated condition such as, but not limited hereto, hyperlipidemia, dyslipidemia, hepatic steatosis, hepatic dysfunction, non-alcoholic fatty liver disease, obesity, diabetes, insulin resistance, metabolic syndrome, arteriosclerosis, hypertension, cardiac angina, heart failure, cardiac infarction, stroke, claudication, retinopathy, eyesight failure, renal failure, electrolyte abnormality, neuropathy, skin ulcer, bulimia, pancreatitis, emmeniopathy, arthritis, gout, cholecystitis, gastroesophageal reflux, Pickwickian syndrome, sleep apnea syndrome, infectious diseases, neoplasia, impaired glucose tolerance, pre-diabetes, hypertriglyceridemia, Cushings Syndrome, lipodystrophy, anemia, nephropathy or cancer.

The present invention also includes a pharmaceutical composition comprising a pharmaceutically acceptable carrier and the compound of Formula (I) described above, and a pharmaceutical composition made by mixing the compound of Formula (I) and a pharmaceutically acceptable carrier.

The invention also features a process for making a pharmaceutical composition comprising mixing the compound described above and a pharmaceutically acceptable carrier.

The invention further provides methods for using the compound or composition of the invention. For example, one embodiment of the invention is a method for treating a condition associated with ACC activity in a subject in need thereof comprising administering to the subject a effective amount of the disclosed compound or the disclosed pharmaceutical compositions.

Other embodiments and features of the invention are disclosed in the following detailed description, examples, and the appended claims.

### DETAILED DESCRIPTION OF THE INVENTION

The present invention relates to the compound of Formula (I):

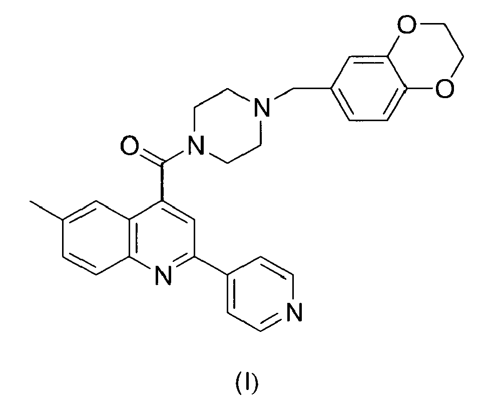

The compound of the present invention is:
Cpd Name
   1 [4-(2,3-Dihydro-benzo[1,4]dioxin-6-ylmethyl)-piperazin-1-yl]-(6-methyl-2-pyridin-4-yl-quinolin-4-yl)methanone

In general, IUPAC nomenclature rules are used herein.

### Compound Forms

The term "about," whether used explicitly or not in reference to a quantitative expression given herein, means that every quantity given herein qualified with the term or otherwise is meant to refer both to the actual given value and the approximation to such given value that would reasonably be inferred based on the ordinary skill in the art, including approximations due to experimental and/or measurement conditions for such given value.

The term "form" means, in reference to the compound of the present invention, such may exist as, without limitation, a salt, stereoisomer, tautomer, crystalline, polymorph, amorphous, solvate, hydrate, ester, prodrug or metabolite form. The present invention encompasses all such compound forms and mixtures thereof.

The term "isolated form" means, in reference to the compound of the present invention, such may exist in an essentially pure state such as, without limitation, an enantiomer, a racemic mixture, a geometric isomer (such as a *cis* or *trans* stereoisomer), a mixture of geometric isomers, and the like. The present invention encompasses all such compound forms and mixtures thereof.

The compound of the invention may be present in the form of pharmaceutically acceptable salts. For use in medicines, the "pharmaceutically acceptable salts" of the compound of this invention refer to non-toxic acidic/anionic or basic/cationic salt forms.

Suitable salt forms include acid addition salts which may, for example, be formed by mixing a solution of the compound according to the invention with a solution of an acid such as acetic acid, adipic acid, benzoic acid, carbonic acid, citric acid, fumaric acid, glycolic acid, hydrochloric acid, maleic acid, malonic acid, phosphoric acid, saccharinic acid, succinic acid, sulphuric acid, tartaric acid, trifluoroacetic acid and the like.

Furthermore when the compound of the present invention carries an acidic moiety, suitable salts thereof may include alkali metal salts, e.g. sodium or potassium salts; alkaline earth metal salts, e.g. calcium or magnesium salts; and salts formed with suitable organic ligands, e.g. quaternary ammonium salts.

Thus, representative salts include the following: acetate, adipate, benzenesulfonate, benzoate, bicarbonate, bisulfate, bitartrate, borate, bromide, calcium, camsylate (or camphosulphonate), carbonate, chloride, clavulanate, citrate, dihydrochloride, edetate, fumarate, gluconate, glutamate, glyconate, hydrabamine, hydrobromine, hydrochloride, iodide, isothionate, lactate, malate, maleate, malonate, mandelate, mesylate, nitrate, oleate, pamoate, palmitate, phosphate/diphosphate, saccharinate, salicylate, stearate, sulfate, succinate, tartrate, tosylate, trichloroacetate, trifluoroacetate and the like.

During any of the processes for preparation of the compound of the present invention, it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene & P.G.M. Wuts, Protective Groups in Organic Synthesis, 3rd Edition, John Wiley & Sons, 1999. The protecting groups may be removed at a convenient subsequent stage using methods known in the art. The scope of the present invention encompasses all such protected compound forms and mixtures thereof.

Furthermore, the compound of the present invention may have one or more polymorph or amorphous crystalline forms and, as such, are intended to be included in the scope of the invention. In addition, the compound may form solvates with water (i.e., hydrates) or common organic solvents (e.g., organic esters such as ethanolate and the like) and, as such, are also intended to be encompassed within the scope of this invention.

### Methods of Use

The present invention provides a quinoline aryl substituted amide compound which is useful as an ACC isozyme inhibitor, inhibiting the production of malonyl CoA by the ACC isozymes: ACC1 and ACC2.

The compound of this invention also shows functional activity by their ability to inhibit the production of malonyl CoA by the ACC isozymes: ACC1 and ACC2.

The instant compound shows the ability to inhibit the production of malonyl CoA by the ACC isozymes: ACC1 and ACC2, and is therefore useful for treating conditions such as hyperlipidemia, dyslipidemia, hepatic steatosis, hepatic dysfunction, non-alcoholic fatty liver disease, obesity, diabetes, insulin resistance, metabolic syndrome, arteriosclerosis, hypertension, cardiac angina, heart failure, cardiac infarction, stroke, claudication, retinopathy, eyesight failure, renal failure, electrolyte abnormality, neuropathy, skin ulcer, bulimia, pancreatitis, emmeniopathy, arthritis, gout, cholecystitis, gastroesophageal reflux, Pickwickian syndrome, sleep apnea syndrome, infectious diseases, neoplasia, impaired glucose tolerance, pre-diabetes, hypertriglyceridemia, Cushings Syndrome, lipodystrophy, anemia, nephropathy or cancers (such as, breast, uterine, ovarian, colon and prostate cancers).

Embodiments of the present invention include a method for treating an ACC mediated condition in a subject in need thereof comprising administering to the subject an effective amount of the compound of Formula (I).

Embodiments of the present invention include a method wherein the compound of Formula (I) is a dual or selective ACC isozyme inhibitor, wherein the ACC isozyme is selected from one or both of ACC1 and ACC2.

Embodiments of the present invention include a use of the compound of Formula (I) in the manufacture of a medicament for treating an ACC mediated condition.

Embodiments of the present invention include a use of the compound of Formula (I) as a medicine.

The compound of Formula (I) has an ACC inhibiting effect and is useful as a therapeutic agent for various ACC mediated disorders, for example, vascular diseases such as hypertension, cardiac angina, heart failure, cardiac infarction, stroke, claudication, diabetic nephropathy, diabetic retinopathy, eyesight failure, electrolyte abnormality and arteriosclerosis; nervous system diseases such as bulimia and diabetic neuropathy; metabolic diseases such as metabolic syndrome, obesity, diabetes, insulin resistance, hyperlipidemia, hypercholesterolemia, hypertriglyceridemia, dyslipidemia, non-alcoholic fatty liver disease, hormone secretion failure, gout and hepatic steatosis; genital diseases such as emmeniopathy and sexual dysfunction; digestive system diseases such as liver dysfunction, pancreatitis, cholecystitis and gastroesophageal reflux; respiratory diseases such as Pickwickian syndrome and sleep apnea syndrome; infectious diseases caused by bacteria, fungi or parasites; malignant neoplasm; and inflammatory diseases such as arthritis and skin ulcer.

Embodiments of the present invention include an ACC mediated condition selected from hyperlipidemia, dyslipidemia, hepatic steatosis, hepatic dysfunction, non-alcoholic fatty liver disease, obesity, diabetes, insulin resistance, metabolic syndrome, arteriosclerosis, hypertension, cardiac angina, heart failure, cardiac infarction, stroke, claudication, retinopathy, eyesight failure, renal failure, electrolyte abnormality, neuropathy, skin ulcer, bulimia, pancreatitis, emmeniopathy, arthritis, gout, cholecystitis, gastroesophageal reflux, Pickwickian syndrome, sleep apnea syndrome, infectious diseases, neoplasia, impaired glucose tolerance, pre-diabetes, hypertriglyceridemia, Cushings Syndrome, lipodystrophy, anemia, nephropathy or cancers (such as, breast, uterine, ovarian, colon and prostate cancers).

Embodiments of the present invention include an ACC mediated condition selected from metabolic syndrome, fatty liver disease, hepatic steatosis, hyperlipidemia, obesity, diabetes, impaired glucose tolerance, pre-diabetes, hypertriglyceridemia, bulimia, malignant neoplasm and infectious diseases.

Embodiments of the present invention include an ACC mediated condition selected from metabolic syndrome, fatty liver disease, hepatic steatosis, hyperlipidemia, obesity, diabetes, impaired glucose tolerance, pre-diabetes and hypertriglyceridemia.

The compound of formula (1) may be administered orally or parenterally, and after formulation into preparations suitable for the intended administration route, it can be used as a therapeutic agent for treating an ACC mediated condition.

The following "diabetes related disorders" are diseases, disorders and conditions that are related to Type 2 diabetes, and therefore may be treated, controlled or in some cases prevented, by treatment with the compound of this invention: (1) hyperglycemia, (2) low glucose tolerance, (3) insulin resistance, (4) obesity, (5) lipid disorders, (6) dyslipidemia, (7) hyperlipidemia, (8) hypertriglyceridemia, (9) hypercholesterolemia, (10) low HDL levels, (11) high LDL levels, (12) atherosclerosis and its sequelae, (13) vascular restenosis, (14) irritable bowel syndrome, (15) inflammatory bowel disease, including Crohn's disease and ulcerative colitis, (16) other inflammatory conditions, (17) pancreatitis, (18) abdominal obesity, (19) neurodegenerative disease, (20) retinopathy, (21) nephropathy, (22) neuropathy, (23) Syndrome X, (24) ovarian hyperandrogenism (polycystic ovarian syndrome), and other disorders where insulin resistance is a component.

In Syndrome X, also known as Metabolic Syndrome, obesity is thought to promote insulin resistance, diabetes, dyslipidemia, hypertension, and increased cardiovascular risk. Therefore, an ACC isozyme inhibitor may also be useful to treat hypertension associated with this condition.

One aspect of the present invention provides a method for the treatment or prevention of disorders, diseases or conditions responsive to the modulation of ACC1 or ACC2 in a subject in need thereof which comprises administering to the subject a therapeutically or prophylactically effective amount of a compound of Formula (I) or a form thereof.

Another aspect of the present invention provides a method for the treatment or prevention of metabolic syndrome, hyperlipidemia, dyslipidemia, non-alcoholic fatty liver disease, obesity, diabetes, bulimia, malignant neoplasm or an infectious disease in a subject in need thereof which comprises administering to said subject a therapeutically or prophylactically effective amount of a compound of Formula (I) or a form thereof.

Another aspect of the present invention provides a method for the treatment of metabolic syndrome, fatty liver disease, hyperlipidemia, obesity, diabetes, impaired glucose tolerance, pre-diabetes, hypertriglyceridemia, bulimia, malignant neoplasm or infectious diseases in a subject in need thereof, which comprises administering to the subject a therapeutically effective amount of a compound of Formula (I) or a form thereof.

Another aspect of the present invention provides a method for the treatment or prevention of diabetes in a subject in need thereof which comprises administering to the subject a therapeutically or prophylactically effective amount of a compound of Formula (I) or a form thereof.

Another aspect of the present invention provides a method for the treatment or prevention of obesity in a subject in need thereof which comprises administering to the subject a therapeutically or prophylactically effective amount of a compound of Formula (I) or a form thereof.

Another aspect of the present invention provides a method for the treatment or prevention of an obesity-related disorder selected from the group consisting of overeating, binge eating, hypertension, elevated plasma insulin concentrations, insulin resistance, hyperlipidemia, endometrial cancer, breast cancer, prostate cancer, colon cancer, kidney cancer, osteoarthritis, obstructive sleep apnea, heart disease, abnormal heart rhythms and arrythmias, myocardial infarction, congestive heart failure, coronary heart disease, sudden death, stroke, polycystic ovary disease, craniopharyngioma, metabolic syndrome, insulin resistance syndrome, sexual and reproductive dysfunction, infertility, hypogonadism, hirsutism, obesity-related gastro-esophageal reflux, Pickwickian syndrome, inflammation, systemic inflammation of the vasculature, arteriosclerosis, hypercholesterolemia, hyperuricaemia, lower back pain, gallbladder disease, gout, constipation, irritable bowel syndrome, inflammatory bowel syndrome, cardiac hypertrophy, left ventricular hypertrophy, in a subject in need thereof which comprises administering to the subject a therapeutically or prophylactically effective amount of a compound of Formula (I) or a form thereof.

Another aspect of the present invention provides a method for the treatment or prevention of hyperlipidemia or dyslipidemia in a subject in need thereof which comprises administering to the subject a therapeutically or prophylactically effective amount of a compound of Formula (I) or a form thereof.

Another aspect of the present invention provides a method for caloric intake in a subject in need thereof which comprises administering to the subject a therapeutically or prophylactically effective amount of a compound of Formula (I) or a form thereof.

Another aspect of the present invention provides a method for reducing food intake in a subject in need thereof which comprises administering to the subject a therapeutically or prophylactically effective amount of a compound of Formula (I) or a form thereof.

Another aspect of the present invention provides a method for increasing satiety in a subject in need thereof which comprises administering to the subject a therapeutically or prophylactically effective amount of a compound of Formula (I) or a form thereof.

Another aspect of the present invention provides a method for reducing appetite in a subject in need thereof which comprises administering to the subject a therapeutically or prophylactically effective amount of a compound of Formula (I) or a form thereof.

The present invention also relates to methods for treating or preventing obesity by administering a compound of Formula (I) or a form thereof, in combination with a therapeutically or prophylactically effective amount of another agent known to be useful to treat or prevent the condition.

The present invention also relates to methods for treating or preventing diabetes by administering a compound of Formula (I) or a form thereof, in combination with a therapeutically or prophylactically effective amount of another agent known to be useful to treat or prevent the condition.

The present invention also relates to methods for treating or preventing hyperlipidemia or dyslipidemia by administering a compound of Formula (I) or a form thereof, in combination with a therapeutically or prophylactically effective amount of another agent known to be useful to treat or prevent the condition.

Another aspect of the present invention provides a pharmaceutical composition comprising a compound of Formula (I) or a form thereof, and a pharmaceutically acceptable carrier.

Yet another aspect of the present invention relates to the use of a compound of Formula (I) or a form thereof, for the manufacture of a medicament useful for the treatment of an ACC mediated disorder in a subject in need thereof.

Yet another aspect of the present invention relates to the use of a compound of Formula (I) or a form thereof, for the manufacture of a medicament useful for the treatment or prevention of metabolic syndrome, hyperlipidemia, dyslipidemia, non-alcoholic fatty liver disease, obesity, diabetes, bulimia, malignant neoplasm or an infectious disease in a subject in need thereof.

Yet another aspect of the present invention relates to the use of a compound of Formula (I) or a form thereof, for the manufacture of a medicament useful for the treatment or prevention of obesity in a subject in need thereof.

Yet another aspect of the present invention relates to the use of a compound of Formula (I) or a form thereof, for the manufacture of a medicament useful for the treatment or prevention of diabetes in a subject in need thereof.

Yet another aspect of the present invention relates to the use of a compound of Formula (I) or a form thereof, for the manufacture of a medicament useful for the treatment or prevention of hyperlipidemia or dyslipidemia in a subject in need thereof.

In a clinical use of the compound of the invention, pharmaceutically-acceptable additives may be added thereto to formulate various preparations in accordance with the intended administration route thereof, and the preparations may be administered.

Various additives generally used in the field of pharmaceutical compositions may be used herein, including, for example, gelatin, lactose, sucrose, titanium oxide, starch, crystalline cellulose, methyl cellulose, hydroxypropylmethyl cellulose, carboxymethyl cellulose, corn starch, microcrystalline wax, white petrolatum, magnesium metasilicate aluminate, anhydrous calcium phosphate, citric acid, trisodium citrate, hydroxypropyl cellulose, sorbitol, sorbitan fatty acid ester, polysorbate, sucrose fatty acid ester, polyoxyethylene, hardened castor oil, polyvinylpyrrolidone, magnesium stearate, palmitoleic acid, light silicic acid anhydride, talc, vegetable oil, benzyl alcohol, gum arabic, propylene glycol, polyalkylene glycol, cyclodextrin, and hydroxypropylcyclodextrin.

Combined with such additives, the compound of the invention may be formulated into various forms of preparations, for example, solid preparations such as tablets, capsules, granules, powders and suppositories; and liquid preparations such as syrups, elixirs and injections. These preparations can be produced in any method known in the field of pharmaceutical compositions. The liquid preparations may be in such a form that is dissolved or suspended in water or in any other suitable medium before use. Especially for injections, the preparation may be dissolved or suspended, if desired, in a physiological saline or glucose solution, and a buffer and a preservative may be added thereto.

The compound of the invention is effective for animals, including humans and other mammals. Any ordinary physician, veterinarian or clinician may readily determine the necessity, if any, of treatment with an instant compound.

When the compound of the invention is, for example, put into clinical use, then its dose and its administration frequency may vary depending on the sex, the age, the body weight and the condition of the patient and on the type and the range of the necessary treatment with the compound. For oral administration, in general, the dose of the compound may be in a range of from about 0.01 to about 100 mg/kg of body weight/day or in a range of from about 0.03 to about 1 mg/kg/day. The oral administration frequency is preferably from one to a few times per day. For parenteral administration, the dose may be in a range of from about 0.00 1 to about 10 mg/kg/day, in a range of from about 0.001 to about 0.1 mg/kg/day or, in a range of from about 0.01 to about 0.1 mg/kg/day. The parenteral administration frequency is preferably from one to a few times per day. For oral administration, the compositions are preferably provided in the form of tablets containing from about 1.0 to about 1000 mg of the active ingredient, particularly 1.0, 5.0, 10.0, 15.0, 20.0, 25.0, 50.0, 75.0, 100.0, 150.0, 200.0, 250.0, 300.0, 400.0, 500.0, 600.0, 750.0, 800.0, 900.0, and 1000.0 mg of the active ingredient for the symptomatic adjustment of the dosage to the patient to be treated. The compound may be administered on a regimen of 1 to 4 times per day, preferably once or twice per day.

When treating an ACC mediated disorder selected from one or more of obesity, diabetes mellitus, hyperlipidemia, dyslipidemia or non-alcoholic fatty liver disease, a therapeutic effect is expected upon administering the compound of the present invention at a daily dosage of from about 0.1 mg to about 100 mg/kg of body weight. The dosing regimen may range from a single daily dose or a divided dose two to six times a day, or in sustained release form. For a large mammal, the total daily dosage may be in a range of from about 1.0 mg to about 1000 mg, or a range of from about 1 mg to about 50 mg. In the case of a 70 kg adult human, the total daily dose will generally be from about 7 mg to about 350 mg. This dosage regimen may be adjusted to provide the optimal therapeutic response.

Ordinary physicians, veterinarians and clinicians may readily determine the effective dose of the pharmaceutical compound necessary to treat, prevent, inhibit, retard or stop the intended disease, and may readily treat the diseased patient with the compound.

The preparation may contain the compound of the invention in an amount in a range of from about 1.0 to about 100 % by weight or, in a range of from about 1.0 to about 60 % by weight of the preparation. The preparation may contain any other therapeutically-effective compound.

In its use, the compound of the invention may be combined with any other therapeutic agents that are useful for the treatment of an ACC mediated disorder.

The term "administration" further means that the individual ingredients to be combined may be administered at the same time or at different times during the treatment period, either as one preparation or as different preparations. Accordingly, the invention should be so interpreted that it encompasses any and every administration mode at the same time or at different times. The range of the combination of the compound of the invention and the other therapeutic agent useful for the above-mentioned disorders encompasses, in principle, all combinations of the compound of the invention and any and every pharmaceutical agent useful for the above-mentioned disorders.

The combination includes not only the composition of the compound of the invention and one other active substance but also the composition of the compound of the invention and two or more other active substances. The scope of possible combinations of a compound of the invention and one, two or more active substances are within the knowledge of one skilled in the art for the treatment of an ACC mediated disorder. For example, for the treatment, management and prevention of metabolic syndrome, a combination of a compound of the invention and one, two or more active substances selected from hypolipidemic agents, lipid lowering agents, and anti-diabetic agents is useful. In particular, a composition that also contains an anti-obesity agent and an anti-hypertension agent, in addition to an anti-diabetic agent and/or a hypolipidemic agent or lipid lowering agent, may exhibit a synergistic effect for treatment of metabolic syndrome.

The compound of the present invention may also be combined with a non-drug therapy such as kinesitherapy, dietetic treatment or radiation therapy.

The compound and the combined compositions of the invention are effective for treating and preventing diabetes.

The term "diabetes" as used herein includes both insulin-dependent diabetes (that is, also known as IDDM, type-i diabetes), and insulin-independent diabetes (that is, also known as NIDDM, type-2 diabetes). Diabetes is characterized by a fasting plasma glucose level of greater than or equal to 126 mg/dl. A diabetic subject has a fasting plasma glucose level of greater than or equal to 126 mg/dl. Prediabetes is characterized by an impaired fasting plasma glucose level (greater than or equal to 110 mg/dl and less than 126 mg/dl), impaired glucose tolerance or insulin resistance. A prediabetic subject is a subject with an impaired fasting plasma glucose level (greater than or equal to-46-mg/dl and less than 126 mg/dl), impaired glucose tolerance (2 hour plasma glucose level of 140 mg/dl and <200 mg/dl) or insulin resistance, resulting in an increased risk of developing diabetes.

The compound and compositions of the invention are useful for treatment of both type-1 and type-2 diabetes. The compound and compositions are especially useful for treatment of type-2 diabetes. The compound and compositions of the invention are especially useful for treatment and/or prevention of pre-diabetes. Also, the compounds and compositions of the invention are especially useful for treatment and/or prevention of gestational diabetes mellitus.

Treatment of diabetes mellitus refers to the administration of a compound or combination of the present invention to treat a diabetic subject. One outcome of the treatment of diabetes is to reduce an increased plasma glucose concentration. Another outcome of the treatment of diabetes is to reduce an increased insulin concentration. Another outcome of the treatment of diabetes is to reduce an increased blood triglyceride concentration and to increase insulin sensitivity. Another outcome of the treatment of diabetes may be enhancing glucose tolerance in a subject with glucose intolerance. Another outcome of the treatment of diabetes is to reduce insulin resistance. Another outcome of the treatment of diabetes is to lower plasma insulin levels, improve glycemic control, particulary in type 2 diabetes, and increase hepatic insulin sensitivity.

Prevention of diabetes mellitus, in particular diabetes associated with obesity, refers to the administration of a compound or combination of the present invention to prevent or treat the onset of diabetes in a subject in need thereof. A subject in need of preventing diabetes includes a prediabetic subject.

The term "hypertension" as used herein includes essential, or primary, hypertension wherein the cause is not known or where hypertension is due to greater than one cause, such as changes in both the heart and blood vessels; and secondary hypertension wherein the cause is known. Causes of secondary hypertension include, but are not limited to obesity; kidney disease; hormonal disorders; use of certain drugs, such as oral contraceptives, corticosteroids, cyclosporin, and the like. The term "hypertension" encompasses high blood pressure, in which both the systolic and diastolic pressure levels are elevated, and isolated systolic hypertension, in which only the systolic pressure is elevated to greater than or equal to 140 mm Hg, while the diastolic pressure is less than 90 mm Hg. One outcome of treatment is decreasing blood pressure in a subject with high blood pressure.

Dyslipidemias or disorders of lipid metabolism, include various conditions characterized by abnormal concentrations of one or more lipids (i.e. cholesterol and triglycerides), and/or apolipoproteins (i.e., apolipoproteins A, B, C and E), and/or lipoproteins (i.e., the macromolecular complexes formed by the lipid and the apolipoprotein that allow lipids to circulate in blood, such as LDL, VLDL and DL).

Dyslipidemia includes atherogenic dyslipidemia. Hyperlipidemia is associated with abnormally high levels of lipids, LDL and VLDL cholesterol, and/or triglycerides. An outcome of the treatment of dyslipidemia, including hyperlipidemia, is to reduce an increased LDL cholesterol concentration. Another outcome of the treatment is to increase a low-concentration of HDL cholesterol. Another outcome of treatment is to decrease very low density lipoproteins (VLDL) and/or small density LDL.

The term "metabolic syndrome", also known as syndrome X, includes three or more of the following symptoms: abdominal obesity, hypertriglyceridemia, low HDL cholesterol, high blood pressure, and high fasting plasma glucose.

The term "obesity" as used herein is a condition in which there is an excess of body fat, and includes visceral obesity. Obesity-induced or obesity-related co-morbidities include, but are not limited to, diabetes, impaired glucose tolerance, insulin resistance syndrome, dyslipidemia, hypertension, hyperuricacidemia, gout, coronary artery disease, myocardial infarction, angina pectoris, sleep apnea syndrome, Pickwickian syndrome, fatty liver disease; cerebral infarction, cerebral thrombosis, transient ischemic attack, orthopedic disorders, arthritis deformans, lumbodynia, emmeniopathy, and infertility. In particular, co-morbidities include: hypertension, hyperlipidemia, dyslipidemia, glucose intolerance, cardiovascular disease, sleep apnea, diabetes mellitus, and other obesity-related conditions.

Treatment of obesity and obesity-related disorders refers to the administration of the compound or combinations of the present invention to reduce or maintain the body weight of an obese subject. One outcome of treatment may be reducing the body weight of an obese subject relative to that subject's body weight immediately before the administration of the compounds or combinations of the present invention. Another outcome of treatment may be decreasing body fat, including visceral body fat.

Another outcome of treatment may be preventing body weight gain. Another outcome of treatment may be preventing the regain of body weight previously lost as a result of diet, exercise, or pharmacotherapy. Another outcome of treatment may be decreasing the occurrence of and/or the severity of obesity-related diseases. The treatment may suitably result in a reduction in food or calorie intake by the subject, including a reduction in total food intake, or a reduction of intake of specific components of the diet such as carbohydrates or fats; and/or the inhibition of nutrient absorption; and/or the inhibition of the reduction of metabolic rate. The treatment may also result in an alteration of metabolic rate, such as an increase in metabolic rate, rather than or in addition to an inhibition of the reduction of metabolic rate; and/or in minimization of the metabolic resistance that normally results from weight loss.

Prevention of obesity and obesity-related disorders refers to the administration of the compound or combinations of the present invention to reduce or maintain the body weight of a subject at risk of obesity. One outcome of prevention may be reducing the body weight of a subject at risk of obesity relative to that subject's body weight immediately before the administration of the compounds or combinations of the present invention. Another outcome of prevention may be preventing body weight regain of body weight previously lost as a result of diet, exercise, or pharmacotherapy. Another outcome of prevention may be preventing obesity from occurring if the treatment is administered prior to the onset of obesity in a subject at risk of obesity. Another outcome of prevention may be decreasing the occurrence and/or severity of obesity-related disorders if the treatment is administered prior to the onset of obesity in a subject at risk of obesity. Moreover, if treatment is commenced in already obese subjects, such treatment may prevent the occurrence, progression or severity of obesity-related disorders, such as, but not limited to, arteriosclerosis, Type 2 diabetes, plycystic ovary disease, cardiovascular diseases, osteoartbritis, dermatological disorders, hypertension,, insulin resistance, hypercholesterolemia, hypertriglyceridemia, and cholelithiasis.

The present invention includes a compound of Formula (I) or a form thereof, wherein the compound is labeled with a ligand for use as a marker, and wherein the ligand is a radioligand selected from deuterium, tritium and the like.

The term "administering," with respect to the methods of the present invention, refers to a means for treating a condition as described herein with a compound of Formula (I) or a form thereof, which would obviously be included within the scope of the invention albeit not specifically disclosed for certain of said compounds.

Such methods include administering an effective amount of compound of Formula (I) or a form thereof at different times during the course of a therapy or concurrently in a combination form. Such methods further include administering an effective amount of said compound with one or more agents at different times during the course of a therapy or concurrently in a combination form.

The term "prodrug" means a compound of Formula (I) or a form thereof that is converted *in vivo* into a functional derivative form that may contribute to therapeutic biological activity, wherein the converted form may be: 1) a relatively active form; 2) a relatively inactive form; 3) a relatively less active form; or, 4) any form which results, directly or indirectly, from such *in vivo* conversions.

Prodrugs are useful when said compound may be either too toxic to administer systemically, absorbed poorly by the digestive tract or broken down by the body before it reaches its target. Conventional procedures for the selection and preparation of suitable prodrug derivatives are described in, for example, "Design of Prodrugs", ed. H. Bundgaard, Elsevier, 1985.

The term "metabolite" means a prodrug form of a compound of Formula (I) or a form thereof converted by *in vivo* metabolism or a metabolic process to a relatively less active functional derivative of said compound.

The term "subject" as used herein, refers to a patient, such as an animal, a mammal or a human, who has been the object of treatment, observation or experiment and is at risk of (or susceptible to) developing an ACC mediated disorder.

The term "effective amount" refers to that amount of a compound of Formula (I) or a form, pharmaceutical composition, medicine or medicament thereof that elicits the biological or medicinal response in a tissue system, animal or human, that is being sought by a researcher, veterinarian, medical doctor, or other clinician, which includes alleviation of the symptoms of the condition being treated.

The effective amount of said compound is from about 0.001 mg/kg/day to about 300 mg/kg/day.

The term "composition" refers to a product containing a compound of Formula (I) or a form thereof, such product comprising specified ingredients in specified amounts, as well as any product which results, directly or indirectly, from such combinations of the specified ingredients in the specified amounts.

The term "medicament" or "medicine" refers to a product containing a compound of Formula (I) or a form thereof. The present invention includes use of such a medicament for treating an ACC mediated disorder.

The term "combination form" refers to the use of a combination product comprising a compound of Formula (I) or a form, pharmaceutical composition, medicine or medicament thereof and at least one therapeutic agent for treating an ACC mediated disorder.

Advantageously, the effective amount of a combination product for treating an ACC mediated disorder may be a reduced amount of either or both the compound or therapeutic agent compared to the effective amount of the compound or therapeutic agent otherwise recommended for treating the condition. Therefore, it is contemplated that the compound is administered to the subject before, during or after the time the agent is administered.

The term "treating" refers, without limitation, to facilitating the eradication of, preventing, ameliorating or otherwise inhibiting the progression of or promoting stasis of an ACC mediated disorder.

The present invention includes a pharmaceutical composition comprising an admixture of a compound of Formula (I) or a form thereof and one or more pharmaceutically acceptable excipients.

The present invention includes a process for making a pharmaceutical composition, medicine or medicament comprising mixing a compound of Formula (I) or a form thereof and an optional pharmaceutically acceptable carrier. The present invention includes a pharmaceutical composition, medicine or medicament resulting from the process of mixing a compound of Formula (I) or a form thereof and an optional pharmaceutically acceptable carrier.

Said pharmaceutical composition, medicine or medicament may take a wide variety of forms to effectuate mode of administration, wherein the mode includes, and is not limited to, intravenous (both bolus and infusion), oral, nasal, transdermal, topical with or without occlusion, and via injection intraperitoneally, subcutaneously, intramuscularly, intratumorally, intracerebrally or intracranially. The composition, medicine or medicament may be in a dosage unit such as a tablet, pill, capsule, powder, granule, sterile parenteral solution or suspension, metered aerosol or liquid spray, drop, ampoule, auto-injector device or suppository for such administration modes.

Pharmaceutical compositions, medicines or medicaments suitable for oral administration include solid forms such as pills, tablets, caplets, capsules (each including immediate release, timed release and sustained release formulations), granules and powders; and, liquid forms such as solutions, syrups, elixirs, emulsions and suspensions. Forms useful for parenteral administration include sterile solutions, emulsions and suspensions. Alternatively, the pharmaceutical composition, medicine or medicament may be presented in a form suitable for once-weekly or once-monthly administration; for example, an insoluble salt of the active compound, such as the decanoate salt, may be adapted to provide a depot preparation for intramuscular injection.

The dosage form (tablet, capsule, powder, injection, suppository, teaspoonful and the like) containing the pharmaceutical composition, medicine or medicament contains an effective amount of the active ingredient necessary to be effective as described above.

An example of a contemplated effective amount for a pharmaceutical composition, medicine or medicament of the present invention may range from about 0.001 mg to about 300 mg/kg of body weight per day. In another example, the range is from about 0.003 to about 100 mg/kg of body weight per day. In another example, the range is from about 0.005 to about 15 mg/kg of body weight per day. The pharmaceutical composition, medicine or medicament may be administered according to a dosage regimen of from about 1 to about 5 times per day.

For oral administration, the pharmaceutical composition, medicine or medicament is preferably in the form of a tablet containing, e.g., 0.01, 0.05, 0.1, 0.5, 1.0, 2.5, 5.0, 10.0, 15.0, 25.0, 50.0, 100, 150, 200, 250 and 500 milligrams of a compound of Formula (I) or a form thereof for the symptomatic adjustment of the dosage to the patient to be treated. Optimal dosages will vary depending on factors associated with the particular patient being treated (e.g., age, weight, diet and time of administration), the severity of the condition being treated, the particular compound being used, the mode of administration and the strength of the preparation. The use of either daily administration or post-periodic dosing may be employed.

### Synthetic Method

The compound of the present invention can be synthesized in accordance with the synthetic scheme described below. The scheme is offered by way of illustration; the invention should not be construed as being limited by the chemical reactions and conditions expressed. The methods for preparing the various starting materials used in the scheme is well within the skill of persons versed in the art. No attempt has been made to optimize the yields obtained in any of the example reactions. One skilled in the art would know how to increase such yields through routine variations in reaction times, temperatures, solvents and/or reagents.

General: ¹H and ¹³C NMR spectra were measured on a Bruker AC-300 (300 MHz) spectrometer using tetramethylsilane and the deuterated solvent respectively as internal standards. Elemental analyses were obtained by Quantitative Technologies Inc. (Whitehouse, New Jersey) and the results were within 0.4% of the calculated values unless otherwise mentioned. Melting points were determined in open capillary tubes with a Mel-Temp II apparatus (Laboratory Devices Inc.) and were uncorrected. Electrospray mass spectra (MS-ESI) were recorded in the positive mode on a Hewlett Packard 59987A spectrometer. High resolution mass spectra (HRMS) were obtained on a Micromass Autospec. E spectrometer by fast atom bombardment (FAB) technique.

The compound of Formula (I) may be synthesized as outlined by the synthetic route illustrated in Scheme A.

### Scheme A

### Method of Synthesis

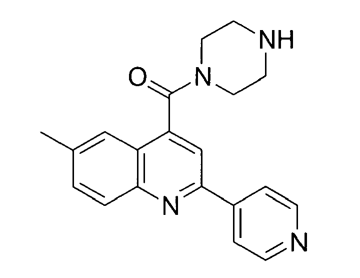

(6-Methyl-2-pyridin-4-yl-quinolin-4-yl)-piperazin-1-yl-methanone:

To a solution of 6-methyl-2-pyridin-4-yl-quinoline-4-carboxylic acid (700 mg, 2.65 mmol) in dimethylformamide (5 mL) was added diimidazol-1-yl-methanone (559 mg, 3.45 mmol). The mixture was stirred at ambient temperature for one h after which piperazine (600 mg, 6.97 mmol) was added in a drop-wise fashion. After stirring at ambient temperature for 16 h, the mixture was concentrated and the resultant residue was purified by flash chromatography (silica gel, 5% methanol in dichloromethane) to give the title intermediate (440 mg, 50% yield). ¹H NMR (400 MHz, CD₃OD) δ (ppm): 8.76(d, J=5.2Hz, 2H), 8.10(d, J=8.8Hz, 1H), 8.06(d, J=5.2Hz, 2H), 7.81 (s, 1H), 7.68(d, J=8Hz, 1H), 7.61(s, 1H), 3.98-3.96(m, 2H), 3.22-3.21(m, 2H), 2.65-2.62(m, 2H), 2.56(s, 3H), 2.34-2.28(m, 2H). LC-MS (ESI+), Calcd. For C₂₀H₂₀N₄O : 333.2 (M+H); Found: 333.4.

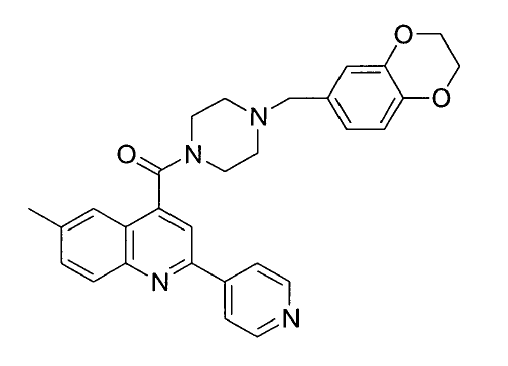

### [4-(2,3-Dihydro-benzo[1,4]dioxin-6-ylmethyl)-piperazin-1-yl]-(6-methyl-2-pyridin-4-yl-quinolin-4-yl)methanone (Compound 1):

To a solution of (6-methyl-2-pyridin-4-yl-quinolin-4-yl)-piperazin-1-yl-methanone (54 mg, 0.16 mmol) in dichlormethane/dimethylformamide (1:1, 2 mL) was added 2,3-dihydrobenzo[1,4]dioxine-6-carboxaldehyde (24.6 mg, 0.15 mmol), sodium triacetoxyborohydride (44.5 mg, 0.21 mmol), and one drop of acetic acid. After stirring at ambient temperature for 16 h, the mixture was concentrated and the resultant residue was purified by flash chromatography (silica gel, 5% methanol in dichloromethane) to give the title compound (32 mg, 45% yield). ¹H NMR (400 MHz, CDCl₃) δ (ppm): 8.78(d, J=5.2Hz, 2H), 8.11(d, J=8.8Hz, 1H), 8.04(d, J=5.2Hz, 2H), 7.77(s, 1H), 7.64(d, J=8Hz, 1H), 7.58(s, 1H), 6.82-6.74(m, 3H), 4.24(s, 4H), 3.98-3.96(m, 2H), 3.45(s, 2H), 3.22-3.21(m, 2H), 2.63-2.61(m, 2H), 2.56(s, 3H), 2.34-2.28(m, 2H). LC-MS (ESI+), Calcd. For C₂₉H₂₈N₄O₃ : 481.2 (M+H); Found 481.3.

Using the procedures detailed in the foregoing example, where appropriate, and suitable starting materials, reagents and reaction conditions known to those skilled in the art, the following compound of Formula (I) may be prepared:

| Cpd | Name | M/Z (M+1)⁺ |
|---|---|---|
| 1 | [4-(2,3-Dihydro-benzo[1,4]dioxin-6-ylmethyl)-piperazin-1-yl]-(6-methyl-2-pyridin-4-yl-quinolin-4-yl)methanone | 481.2 |

### Biological Examples

The ability of the compound of the present invention to treat an ACC mediated condition was determined using the following procedures.

### Example 1

### Expression and Purification of the Carboxyterminus (CT) of ACC1 and ACC2

The 6His.FLAG.TEV Human ACC2 (amino acids 1685-2458) (Egea Biosciences, San Diego, CA) and 6His.FLAG. TEV. Human ACC1 (amino acids 1603-2383) (Egea Biosciences, San Diego, CA.) genes were synthesized and subcloned into the pENTR11 vector. Transfection-grade DNA was purified using the QIAwell^{®} Kit from Qiagen. The LR reaction was performed overnight and then transfected into Sf9 cells using a BaculoDirect^{®} Baculovirus Expression System (Invitrogen). The P₀ virus was collected 4 days post transfection and used for another round of virus amplification. The resulting P₁ virus and cells were collected 3 days post-infection.

A P₂ virus was expanded in Sf9 cells to generate a high titer P₃ stock for recombinant protein expression by infecting Sf9 cells in suspension at an MOI of 0.3 and harvesting the virus after 72 hours. A cell paste for protein purification was obtained by infecting Sf9 cells at a density of 1.5 x10⁶/mL with an MOI of 1.

Cultures were maintained at 27 °C for about 65 hours to about 72 hours, with shaking at 140 rpms. Cells were harvested by centrifugation at 1000x g for 10 minutes at 4 °C. Following collection, cell pellets were washed in PBS with broad range protease inhibitors and stored at -80 °C. Samples were saved for SDS-PAGE and Western blot analysis.

Frozen cells were thawed and resuspended in 50 mM Tris buffer (at pH 8.0, containing 400 mM NaCl, 5% glycerol, 0.05% β-mercaptoethanol (BME), 20 mM imidazole, 2.5 U/mL benzonase and 1 kU/mL rLysozyme) in a 2X complete EDTA-free protease inhibitor cocktail (Roche). Resuspended cells were dounce homogenized and mechanically lysed with a microfluidizer processor (Microfluidics) at 18,000 psi.

The lysate was clarified by centrifugation at 43,000 g for 1 hour. All of the following purification steps were performed on a ÄKTAxpress System^{®} (GE Healthcare) at 4°C and were fully automated. The supernatant was loaded onto a 1 mL HiTrap^{®} crude column (GE Healthcare) and the resin was washed with 30 column volumes of buffer A (50 mM Tris buffer pH 8.0, 400 mM NaCl, 5% glycerol, 0.05% BME and 20 mM imidazole).

On-column cleavage of the histidine tag was performed by injecting 96 ug of TEV S219V protease/mg (of ACC2 CT. The product was incubated at 4 °C for 20 hours. The cleaved ACC2 was eluted in buffer A and loaded directly onto a HiLoad^{®} 16/60 Superdex 200 column (GE Healthcare), preequilibrated with 25 mM Tris Buffer (at pH 8.0, 200 mM NaCl, 5% Glycerol, 5 mM DTT). Fractions containing ACC2 CT, assayed by SDS-PAGE, were pooled. For cocrystallization, the compound was added in a 1:2 molar ratio of protein:compound and incubated overnight at 4°C. The complex was concentrated to a final protein concentration of 7 mg/mL using an 30 kDa cut-off Ultrafree^{®} membrane (Millipore, Billerica, MA) and was then ready for crystallization.

### Thermoflour Measurement of Compound Binding to ACC1 and 2 CT Domains

ThermoFluor (TF) experiments were carried out using instruments available from Johnson & Johnson Pharmaceutical Research & Development, LLC, Exton, PA. The TF dye used in all experiments was 1,8-ANS (Invitrogen: Catalog No. A-47). For ACC1, final TF assay conditions were 0.05 mg/mL of ACC1 in a solution with 60 uM ANS, 100 mM NaCl and 0.0025% Tween-20 in 50 mM PIPES at pH 7.0. For ACC2, final TF assay conditions included 0.065 mg/mL of ACC2 in a solution with 60 uM ANS, 100 mM NaCl and 0.0025% Tween-20 in 50 mM PIPES at pH 7.0. Both proteins were routinely tested in the assay both with and without 2 mM Acetyl Coenzyme A (Sigma-Aldrich: A2056).

The compound was arranged in a pre-dosed mother plate (Greiner Bio-one: Catalog No. 781280) and serially diluted in 100% DMSO across each of 11 columns within a series. Columns 12 and 24 contained only DMSO and were used for vehicle reference. The compound aliquots (46 nL) were robotically predispensed directly into 384-well black assay plates (Abgene: Catalog No. TF-0384/k) using the Hummingbird^{®} liquid handler (Genomics Solutions) and the protein and dye solutions were added to achieve the final assay volume of µL. The assay solutions were overlayed with 1 µL of silicone oil (Fluka, type DC 200: 85411) to prevent evaporation.

The bar-coded assay plates were robotically loaded onto a thermostatically controlled PCR-type thermal block and then heated at about 25°C to about 90 °C at a ramp-rate of 1 °C/min for all experiments. Fluorescence was measured by continuous illumination with UV light (Hamamatsu LC6) supplied via fiber optic and filtered through a band-pass filter (380-400 nm; >6 OD cutoff). Fluorescence emission of the entire 384-well plate was detected by measuring light intensity using a CCD camera (Sensys, Roper Scientific) filtered to detect 500 ± 25 nm, resulting in simultaneous and independent readings of all 384 wells. A single image with 20 sec exposure time was collected at each temperature and the sum of the pixel intensity in a given area of the assay plate was recorded against temperature. Reference wells contained protein without compounds. K_{d} calculations were done according to the procedure described in Matulis D, Kranz JK, Salemme FR and Todd MJ, Thermodynamic stability of carbonic anhydrase: measurements of binding affinity and stoichiometry using ThermoFluor, Biochem., 2005, 44, 5258-5266.

The IC₅₀ data derived from the best curve fit is shown in Table 1.

**Table 1**

| ACC1 and ACC2 TF K_{d} (µM) | | |
|---|---|---|
| **Cpd** | **ACC2** | **ACC1** |
| **1** | 0.40 | 0.97 |

### Example 2

### ApoB Secretion from HepG2 cells

HepG2 cells (American Type Culture Collection: Catalog No. #HB-8065) are maintained in Growth Media consisting of DMEM with 4.5 g/L glucose (Cellgro: Catalog No. #10-013-CV), supplemented with 10% fetal bovine serum (FBS) (Cellgro: Catalog No. #35-011-CV) and 100 U/mL each penicillin and streptomycin (Cellgro: Catalog No. #30-002-CI). For the assay, cells are plated @ ∼10,000 cells/well in white, clear-bottom, 96-well plates (Costar: Catalog No. #3610) and incubated in a humidified 5% CO₂ atmosphere at 37 °C. The next day, Growth Media is replaced with RPMI 1640 without glucose (Cellgro: Catalog No. #10-043-CV), supplemented with 10% charcoal/dextran treated FBS (Hyclone: Catalog No. #SH30068.02) and 100 U/mL each penicillin and streptomycin (starvation media). The cells are maintained overnight in a humidified 5% CO₂ atmosphere at 37 °C. On the day of the assay, the starvation media is removed from each well and replaced with fresh assay media (DMEM with 4.5g/L glucose, supplemented with 10% charcoal/dextran treated FBS and 100 U/mL each penicillin and streptomycin) and samples of 0.5% Me₂SO both with and without test compound. Twenty-four hours later, the growth medium is collected and assessed for ApoB content.

High-binding white polystyrene plates (Costar: Catalog No. #3922) are coated with 100 µL of 1 µg/mL affinity-purified polyclonal goat anti-human ApoB capture antibody (Rockland Product No. # 600-101-111) and incubated overnight at 4 °C. The capture antibody is removed and 200 µL Pierce SuperBlock^{®} (Pierce, Rockland, IL, Cat # 37515) is added to each well and the plate is incubated for one hour at 37 °C. The SuperBlock^{®} is removed and the plate is washed 3x with 200 µL PBS, containing 0.01% Tween 20 (PBS-T). Samples of 40 µL are added to the wells with 60 µL PBS-T and the plate is incubated again for one hour at 37 °C.

The samples are removed and the plate is washed again 3x with 200 µL PBST, then 100 µL of 1/10,000 HRP-conjugated, affinity-purified polyclonal sheep anti-human ApoB detection antibody (BioDesign International Product No. # K90086P) is added to each well and the plate is incubated for one hour at 37 °C. The detection antibody is removed from the wells and the plate is washed 5x with 200 µL PBST, then 100 µL of Pierce SuperSignal^{®} is added to each well and incubated for 1 minute at room temperature on the plate shaker. The plate is read for one second in an Orion Microplate Luminometer^{®}. (Berthold Technologies, Düsseldorf, Germany) Data is curve fit with Prism4 software using a sigmoidal dose response equation with variable slope and constraints of 100% maximum and shared bottom value for all data. The IC₅₀ data derived from the best curve fit is shown in Table 2. The symbol "∼" means the value shown is approximate.

**Table 2**

| ApoB IC₅₀ (µM) | |
|---|---|
| **Cpd** | **IC₅₀** |
| **1** | 8 |

Throughout this application, various publications are cited. The disclosure of these publications is hereby incorporated by reference into this application to describe more fully the state of the art to which this invention pertains.

While the foregoing specification teaches the principles of the present invention, with an example provided for the purpose of illustration, it will be understood that the practice of the invention encompasses all of the usual variations, adaptations and modifications as come within the scope of the following claims and their equivalents.

## Claims

1. A compound of Formula (I): 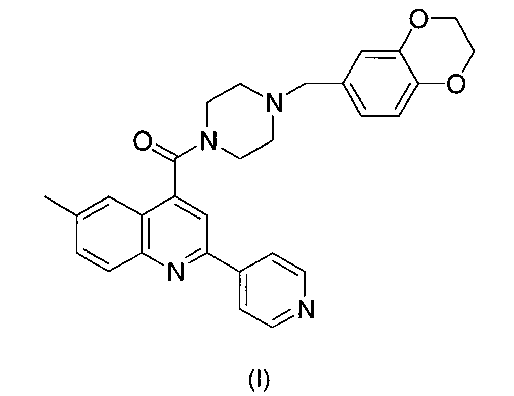 or a pharmaceutically acceptable form thereof.

2. A compound consisting of:
[4-(2,3-Dihydro-benzo[1,4]dioxin-6-ylmethyl)-piperazin-1-yl]-(6-methyl-2-pyridin-4-yl-quinolin-4-yl)methanone.

3. A pharmaceutical composition comprising a compound of claim 1 and a pharmaceutically acceptable carrier or excipient.

4. A method for treating an ACC isozyme mediated disorder in a subject in need thereof comprising a step of administering an effective amount of the compound of claim 1.

5. The method of claim 4, wherein the ACC mediated condition is selected from vascular diseases such as hypertension, cardiac angina, heart failure, cardiac infarction, stroke, claudication, diabetic nephropathy, diabetic retinopathy, eyesight failure, electrolyte abnormality and arteriosclerosis; nervous system diseases such as bulimia and diabetic neuropathy; metabolic diseases such as metabolic syndrome, obesity, diabetes, insulin resistance, hyperlipidemia, hypercholesterolemia, impaired glucose tolerance, pre-diabetes, hypertriglyceridemia, dyslipidemia, non-alcoholic fatty liver disease, hormone secretion failure, gout and hepatic steatosis; genital diseases such as emmeniopathy and sexual dysfunction; digestive system diseases such as liver dysfunction, pancreatitis, cholecystitis and gastroesophageal reflux; respiratory diseases such as Pickwickian syndrome and sleep apnea syndrome; infectious diseases caused by bacteria, fungi or parasites; malignant neoplasm; and inflammatory diseases such as arthritis and skin ulcer.

6. The method of claim 5, wherein the condition is selected from metabolic syndrome, fatty liver disease, hepatic steatosis, hyperlipidemia, obesity, diabetes, impaired glucose tolerance, pre-diabetes, hypertriglyceridemia, bulimia, malignant neoplasm and infectious diseases.

7. The method of claim 6, wherein the condition is selected from metabolic syndrome, fatty liver disease, hepatic steatosis, hyperlipidemia, obesity, diabetes, impaired glucose tolerance, pre-diabetes and hypertriglyceridemia.

8. The method of claim 4, wherein the effective amount is from about 0.001 mg/kg/day to about 300 mg/kg/day.

9. Use of the compound of claim 1 in the manufacture of a medicament for treating an ACC isozyme mediated disorder.

10. A compound of claim 1 for use in a method for treating an ACC isozyme mediated disorder comprising administering an effective amount of the compound.

11. The use of claim 9 or the compound of claim 10, wherein the ACC mediated disorder is selected from vascular diseases such as hypertension, cardiac angina, heart failure, cardiac infarction, stroke, claudication, diabetic nephropathy, diabetic retinopathy, eyesight failure, electrolyte abnormality and arteriosclerosis; nervous system diseases such as bulimia and diabetic neuropathy; metabolic diseases such as metabolic syndrome, obesity, diabetes, insulin resistance, hyperlipidemia, hypercholesterolemia, impaired glucose tolerance, pre-diabetes, hypertriglyceridemia, dyslipidemia, non-alcoholic fatty liver disease, hormone secretion failure, gout and hepatic steatosis; genital diseases such as emmeniopathy and sexual dysfunction; digestive system diseases such as liver dysfunction, pancreatitis, cholecystitis and gastroesophageal reflux; respiratory diseases such as Pickwickian syndrome and sleep apnea syndrome; infectious diseases caused by bacteria, fungi or parasites; malignant neoplasm; and inflammatory diseases such as arthritis and skin ulcer.

12. The use of claim 11 or the compound of claim 11, wherein the disorder is selected from metabolic syndrome, fatty liver disease, hepatic steatosis, hyperlipidemia, obesity, diabetes, impaired glucose tolerance, pre-diabetes, hypertriglyceridemia, bulimia, malignant neoplasm and infectious diseases.

13. The use of claim 12 or the compound of claim 12, wherein the disorder is selected from metabolic syndrome, fatty liver disease, hepatic steatosis, hyperlipidemia, obesity, diabetes, impaired glucose tolerance, pre-diabetes and hypertriglyceridemia.

14. The compound of claim 10, wherein the effective amount is from about 0.001 mg/kg/day to about 300 mg/kg/day.
